# EUROPÄISCHE PATENTSCHRIFT

(11) **EP 3 494 932 B1**
(45) Veröffentlichungstag und Bekanntmachung des Hinweises auf die Patenterteilung: **25.01.2023**
(21) Anmeldenummer: 18211483.5
(22) Anmeldetag: 11.12.2018
(51) Int. Cl.: A61F 2/50, A61F 2/76, A61F 2/80, G06T 7/00, G06T 17/00

(54) **VERFAHREN ZUR ERSTELLUNG EINES MODELLS FÜR EINEN GLIEDMASSENSTUMPF ZUR HERSTELLUNG EINES PROTHESENSCHAFTS**
METHOD FOR PRODUCING A MODEL FOR A LIMB STUMP FOR MAKING A PROSTHESIS SHAFT
PROCÉDÉ DE PRODUCTION D'UN MODÈLE POUR UN MOIGNON DE MEMBRE DESTINÉ À FABRIQUER UNE PROTHÈSE

(30) Priorität: 11.12.2017 DE 102017222363
(43) Veröffentlichungstag der Anmeldung: 12.06.2019
(73) Patentinhaber: Pohlig GmbH, 83278 Traunstein (DE)
(72) Erfinder: Pohlig, Kurt, 83278 Traunstein (DE); Schäfer, Michael, 83278 Traunstein (DE)
(74) Vertreter: Pfenning, Meinig & Partner mbB

(56) Entgegenhaltungen:
- EP-A1- 1 843 291
- EP-A1- 2 873 393
- WO-A1-2010/006728
- ERGIN T\N]KTONUK ET AL: "NONLINEAR ELASTIC MATERIAL PROPERTY ESTIMATION OF LOWER EXTREMITY RESIDUAL LIMB TISSUES", 1. März 2003 (2003-03-01), IEEE TRANSACTIONS ON NEURAL SYSTEMS AND REHABILITATIONENGINEERING, IEEE SERVICE CENTER, NEW YORK, NY, US, PAGE(S) 43 - 53, XP011078111, ISSN: 1534-4320 * Seite 46, linke Spalte, Absatz 3 - Seite 47, rechte Spalte, Absatz 1 *

## Beschreibung

Die vorliegende Erfindung betrifft ein Verfahren zur Erstellung eines Modells für einen Gliedmaßenstumpf zur Herstellung eines Prothesenschafts und ein Verfahren zur Herstellung des Prothesenschafts sowie einen Prothesenschaft.

Bei der Herstellung einer Prothese für einen Gliedmaßenstumpf ist es wesentlich, die Prothese optimal an den Gliedmaßenstumpf anzupassen. Hierzu wird, wie im Folgenden anhand eines Beispiels eines Oberschenkelstumpfes beschrieben wird, herkömmlicherweise zuerst der Oberschenkelumfang auf verschiedenen Höhen (d.h. an Umfangs-Linien in etwa oder vollständig senkrecht zur Längserstreckung des Oberschenkelknochens) vermessen. Dies kann beispielsweise im Abstand von 3 cm parallel zueinander erfolgen. Zusätzlich wird auf diesen Höhen eine Bestimmung des Maßes der Eindellung des Gewebes ermittelt, die auftritt, wenn das Maßband einer definierten Zugkraft unterworfen wird. Alternativ dazu kann auch die Zugkraft gemessen werden, die zur Erreichung eines bestimmten Maßes an Einschnürung erforderlich ist. Der Orthopädie-Techniker ermittelt aus dem Oberschenkelumfang und der Zugkraft/Einschnürungsmessung das sogenannte Reduktionsmaß (RM). Dieses Reduktionsmaß wird dann vom Orthopädie-Techniker zur Bemaßung und Konstruktion des Schaftes der Prothese verwendet, so dass der Prothesenschaft optimal an den vermessenen Oberschenkelstumpf und dessen Eigenschaften angepasst ist.

Problematisch bei diesem Stand der Technik ist, dass die Bestimmung des Reduktionsmaßes eine subjektive Komponente bei der Messung des Oberschenkelumfangs und/oder bei der Zugkraftmessung enthält. Diese ist letztlich abhängig von der Person, die das Reduktionsmaß misst und damit nicht vollständig reproduzierbar. Das herkömmliche, händische Verfahren zur Bemaßung eines Prothesenschaftes ermöglicht es also nicht, vergleichbare oder gar einheitliche hohe Standards bei der Konstruktion des Schaftes zu gewährleisten.

Als weitere Möglichkeit des Standes der Technik zur Bemaßung des Prothesenschaftes wird von dem Gliedmaßenstumpf ein Gipsabdruck genommen, der dann anschließend bei der Schaftformung verwendet wird. Auch dieses Verfahren ist stark von der ausführenden Person abhängig und nicht gesichert reproduzierbar.

Als weiteres Verfahren im Stand der Technik wird die Computertomografie oder Magnetresonanztomografie eingesetzt. Mit diesen lassen sich dreidimensionale Daten über Größe, Form und Lage von Knochen und Weichteilen ermitteln. Aus diesen Daten kann ebenfalls die Bemaßung des Schaftes ermittelt werden. Nachteilig an diesem Verfahren sind der hohe gerätetechnische Aufwand und die hohen Kosten, die für die tomografische Bildgebung erforderlich sind. Weiterhin hat dieses Verfahren den Nachteil, dass bei diesen tomografischen bildgebenden Verfahren der Gliedmaßen-Stumpf nur im entspannten Zustand, d.h. ohne Muskelkontraktion, erfasst werden kann.

Weiterhin wird mit den im Stand der Technik bekannten Verfahren zur Herstellung eines Prothesenschaftes trotz der individuellen Ausmessung des Gliedmaßenstumpfs unter Berücksichtigung der Kompressibilität des Stumpfgewebes und der Bestimmung der Reduktionsmaße keine sicher reproduzierbaren Prothesenschäfte mit optimaler Passform erreicht. Zum erreichen der optimalen Passform ist daher eine Anfertigung mehrerer Probeschäfte sowie eine Nachbearbeitung der Probeschäfte notwendig.

In der Druckschrift EP 2 873 393 A1 wird ein Verfahren zur Bestimmung der Bemaßung eines Prothesenschaftes und ein Verfahren zur Herstellung einer Prothese für einen Gliedmaßenstumpf angegeben, das schnell, kostengünstig und in hohem Grade reproduzierbar durchgeführt werden kann.

In der Druckschrift WO 2010/006728 werden ein Verfahren, ein System, eine Vorrichtung und ein Computerprogramm zur Erzeugung und Modifizierung eines 3D-Schaft-Stumpfmodells zur Herstellung eines Prothesenschaftes zur Verbindung eines einen Stumpf bildenden Körperteils mit einer Prothese beschrieben.

In der Druckschrift EP 1 843 291 A1 wird ein Verfahren zur Erstellung eines Prothesenschafts für einen Extremitäten-Stumpf eines Patienten bereitgestellt. Das Verfahren umfasst Separieren der Anteile von Knochen-, Muskel- und Fettgewebe aus einer dreidimensionalen Darstellung des Stumpfs, Bestimmen einer Ziel-Kompression des Stumpfs auf der Grundlage des Gewichts des Patienten und der Außenfläche des Stumpfs, Bestimmen einer Vielzahl von Querschnitten des Stumpfs, basierend auf der Ziel-Kompression und einer jeweils vorgegebenen Kompressibilität der Anteile von Muskel- und Fettgewebe, und Erstellen eines stetigen dreidimensionalen Modells des Prothesenschafts aus der Vielzahl von Querschnitten. Die entsprechende Vorrichtung der Erfindung umfasst eine Einrichtung, die angepasst ist für ein Separieren der Anteile von Knochen-, Muskel- und Fettgewebe aus einer dreidimensionalen Darstellung des Stumpf, eine Einrichtung, die angepasst ist für ein Bestimmen einer Ziel-Kompression des Stumpfs basierend auf dem Gewicht des Patienten und der Außenfläche des Stumpfs, eine Einrichtung, die angepasst ist für ein Bestimmen einer Vielzahl von Querschnitten des Stumpfs, basierend auf der Ziel-Kompression und einer jeweils vorgegebenen Kompressibilität der Anteile von Muskel- und Fettgewebe, und eine Einrichtung, die angepasst ist für ein Erstellen eines stetigen dreidimensionalen Modells des Prothesenschafts aus der Vielzahl von Querschnitten.

Aufgabe der vorliegenden Erfindung ist es daher ein Verfahren zur Erstellung eines Modells für einen Gliedmaßenstumpf zur Herstellung eines Prothesenschaftes und ein Verfahren zur Herstellung des Prothesenschaftes zur Verfügung zu stellen, mit dem auf Anhieb eine möglichst optimale Passform des Prothesenschafts erreicht werden kann und das schnell und in hohem Grade reproduzierbar durchgeführt werden kann. Weiterhin ist es eine Aufgabe der Erfindung einen Prothesenschaft mit guter Passform, der schnell und reproduzierbar herstellbar ist, zur Verfügung zu stellen.

Diese Aufgabe wird durch ein Verfahren zur Erstellung eines Modells für einen Gliedmaßenstumpf zur Herstellung eines Prothesenschafts gemäß Anspruch 1 sowie ein Verfahren zur Herstellung des Prothesenschafts nach Anspruch 12 gelöst. Vorteilhafte Weiterbildungen der erfindungsgemäßen Verfahren werden in den abhängigen Ansprüchen gegeben.

Das erfindungsgemäße Verfahren zur Erstellung eines Modells, insbesondere eines digitalen Modells, zur Herstellung eines Prothesenschafts für einen Gliedmaßenstumpf umfasst einen Erfassungsschritt, in dem die Oberfläche des Gliedmaßenstumpfes erfasst, insbesondere digital erfasst, wird, einen Bestimmungsschritt, in dem eine Gewebekonsistenz des Gliedmaßenstumpfs bestimmt wird und einen Modellerstellungsschritt, in dem ein Modell, insbesondere ein digitales Modell, des Gliedmaßenstumpfes auf Grundlage von in dem Erfassungsschritt erfassten Daten erstellt wird. Das erfindungsgemäße Verfahren zeichnet sich dadurch aus, dass in einem Einteilungsschritt in dem Modell eine Bezugsebene festgelegt wird, das Modell in mehrere parallel zur Bezugsebene verlaufende Volumenscheiben eingeteilt wird, in einem Reduktionsschritt basierend auf der bestimmten Gewebekonsistenz ein reduzierter Volumenwert von mindestens einer Volumenscheibe ermittelt wird und in einem Modifikationsschritt ein Volumen der mindestens einen Volumenscheibe gemäß dem reduzierten Volumenwert unter Beibehaltung einer Dicke der Volumenscheibe und einer Form einer parallel zur Bezugsebene verlaufenden Querschnittskontur der Volumenscheibe reduziert wird und das Modell entsprechend der volumenreduzierten Volumenscheibe angepasst wird.

Durch eine Reduktion des Volumens mindestens einer Volumenscheibe wird eine deutliche Verbesserung in der Passform des späteren Prothesenschafts erreicht. Insbesondere wird eine Verbesserung in der Passform im Vergleich zu einer Reduktion der Umfangsmaße eines Gliedmaßenstumpfmodells zur Herstellung eines Prothesenschafts erreicht. Auf diese Weise können gut angepasste Prothesenschäfte direkt hergestellt werden, wodurch eine geringere Anzahl an sukzessiv angepassten Probeschäften nötig ist und sich somit eine erhebliche Verkürzung der Herstellungszeit ergibt.

Der Erfassungsschritt kann vorzugsweise einen Scanschritt umfassen, in dem die Oberfläche des Gliedmaßenstumpfes gescannt und erfasst, insbesondere digital erfasst, wird.

In einer vorteilhaften Ausgestaltung der Erfindung können in dem Reduktionsschritt mehrere reduzierte Volumenwerte für mehrere Volumenscheiben ermittelt werden, in dem Modifikationsschritt ein Volumen der mehreren Volumenscheiben gemäß den mehreren reduzierten Volumenwerten reduziert werden und das Modell entsprechend den volumenreduzierten Volumenscheiben angepasst werden.

In einer bevorzugten Ausgestaltung der Erfindung erstreckt sich die Bezugsebene im Wesentlichen senkrecht zu einer Längsachse des Gliedmaßenstumpfes. Dabei kann die Längsachse des Gliedmaßenstumpfs insbesondere eine Längsmittelachse des Gliedmaßenstumpfes sein. Ferner ist es auch denkbar, dass die Längsachse nicht durchgängig gerade ist, sondern eine Knickstelle aufweist. Dies kann vorteilhaft sein, wenn sich der Stumpf nicht gerade erstreckt, sondern insbesondere in einem distalen Bereich abgewinkelt geformt ist.

Weiterhin kann das erfindungsgemäße Verfahren einen Sonografieschritt umfassen, in dem eine Verteilung verschiedener Gewebearten innerhalb des Gliedmaßenstumpfes bestimmt wird. Die verschiedenen Gewebearten können insbesondere unterschiedliche Gewebekonsistenzen aufweisen. In dem Reduktionsschritt kann dann der eine oder die mehreren reduzierten Volumenwerte unter anderem basierend auf den im Sonografieschritt erfassten Daten ermittelt werden.

Die Volumenscheiben können eine einheitliche oder unterschiedliche Dicke aufweisen. Insbesondere kann die Dicke der Volumenscheiben konstant sein oder innerhalb einer Volumenscheibe variieren. Die Dicke der Volumenscheiben kann ≥ 1cm und/oder ≤5 cm, vorzugsweise 3 cm, betragen.

In dem Modellerstellungsschritt kann das Modell weiterhin auf Grundlage eines Knochenmodells für den Gliedmaßenstumpf erstellt werden. Das Knochenmodell für den Gliedmaßenstumpf kann auch nach dem Modellerstellungsschritt in das Modell eingepasst werden. Besonders bevorzugt ist es, wenn das Knochenmodell nach dem Modellerstellungsschritt und vor dem Reduktionsschritt in das Modell eingefügt wird. Das Knochenmodell kann vorteilhafterweise ein standardisiertes oder individuelles Knochenmodell sein, welches in seiner Größe, Position und/oder seinen Proportionen an den Gliedmaßenstumpf anpassbar ist.

Zusätzlich kann eine Kompressibilität und/oder Verschieblichkeit eines Stumpfgewebes gemessen werden, und die Kompressibilität und/oder Verschieblichkeit bei der Ermittlung der reduzierten Volumenwerte berücksichtigt werden. Insbesondere kann die Gewebekonsistenz, die Kompressibilität und/oder Verschieblichkeit des Stumpfgewebes in einer oder mehreren der Volumenscheiben mittels einer Zugbandmessung bestimmt werden. Die Durchführung der Zugbandmessung für mehrere Volumenscheiben kann die reduzierten Volumenwerte und damit die Passform des späteren Prothesenschaftes weiter optimieren.

In dem Modifikationsschritt kann weiterhin eine Konturfläche des Modells des Gliedmaßenstumpfs in Abhängigkeit von einer Stärke der Reduktion der Volumenwerte zu einem Stumpfende hin verlängert werden. Dies ist vorteilhaft, da ein später auf Grundlage des Modells hergestellter Prothesenschaft in der Lage ist, durch den eng anliegenden Sitz des Prothesenschaftes nach unten verdrängtes Stumpfgewebe aufzunehmen und so Druckstellen zu vermeiden.

Vorzugsweise beträgt eine Verlängerung der Konturfläche ≥ 3 mm und/oder ≤ 20 mm, insbesondere >_ 5 mm und/oder < 15 mm. Im Fall einer weichen Gewebekonsistenz ist es vorteilhaft, die Konturfläche um ≥ 13 mm und/oder ≤ 20 mm, insbesondere um 15 mm, zu verlängern. Im Fall einer mittleren Gewebekonsistenzrs ist es vorteilhaft, die Konturfläche um ≥ 8 mm und/oder≤ 12 mm, insbesondere um 10 mm, zu verlängern. Im Fall einer festen Gewebekonsistenz ist es vorteilhaft, die Konturfläche um ≥ 3 mm und/oder ≤ 7 mm, insbesondere um 5 mm zu verlängern. Besonders bevorzugt ist es weiterhin, wenn hierzu das Volumen der distalen Volumenscheibe am Stumpfende entsprechend vergrößert wird.

Weiterhin können in dem Modifikationsschritt eine oder mehrere der Volumenscheiben parallel zur Bezugsebene und vorzugsweise innerhalb einer Konturfläche des Modells des Gliedmaßenstumpfs nach biomechanischen Aspekten und/oder zur Optimierung der Stützfähigkeit des Prothesenschaftes verschoben werden. Besonders vorteilhaft ist es, wenn eine oder mehrere der reduzierten Volumenscheiben bis an einen lateralen Außenrand des Modells des Gliedmaßenstumpfes verschoben werden, sodass die verschobenen Volumenscheiben bündig mit dem lateralen Außenrand sind. In einer weiteren vorteilhaften Variante können eine oder mehrere der Volumenscheiben derart senkrecht zur Längsmittelachse verschoben werden, dass insbesondere die oberste (proximale) Volumenscheibe einen Abstand zu dem lateralen Außenrand des Modells von ≥ 5 mm und/oder ≤ 15 mm, insbesondere von 10 mm, aufweist und zu einem medialen Innenrand des Modells einen Abstand von >_ 15 mm und/oder ≤ 25 mm, insbesondere von 20 mm, aufweist. Durch eine Verschiebung der Volumenscheiben nach lateral kann eine Anlage des Gliedmaßenstumpfes und/oder eine Kraftableitung über ein weiches Gewebe und/oder Muskeln in einem medialen Bereich des späteren Prothesenschaftes verbessert werden.

Weiterhin ist es vorteilhaft, wenn in dem Modell nach der Volumenreduktion bzw. nach dem Verschieben der volumenreduzierten Volumenscheiben die Volumenscheiben miteinander verbunden und die Konturfläche des Modells geglättet wird.

In dem Modifikationsschritt kann ein distaler Anteil des Modells des Gliedmaßenstumpfs zur Entlastung des distalen Femurendes, vorzugsweise nach lateral, verkippt werden. Ein ähnlicher Entlastungseffekt kann auch durch ein Verschieben der distal gelegenen Volumenscheiben in Richtung der Verkippung erreicht werden.

Des Weiteren können in dem Modifikationsschritt entlang einer Umfangsrichtung einer Konturfläche des Modells Stabilisatoren zur Fixierung des Prothesenschafts in einer Umfangsrichtung eingefügt werden. Um die Positionierung der Stabilisatoren zu vereinfachen, können vor dem Erfassungsschritt an dem Gliedmaßenstumpf Markierungen, beispielsweise mittels selbstklebender Landmarks und/oder Hautmarker, für die Stabilisatoren angebracht werden. Diese Markierungen können miterfasst oder mitgescannt und dann zur digitalen Positionierung der Stabilisatoren verwendet werden. Weiterhin können mittels einer Sonografiemessung Muskellücken erfasst und anschließend in dem Modell zur Positionierung der Stabilisatoren markiert werden.

Die Stabilisatoren können vorzugsweise länglich ausgebildet sein, wobei eine Längsachse der Stabilisatoren im Wesentlichen parallel zur Längsachse des Gliedmaßenstumpfes verläuft. Eine Größe und/oder Dicke der Stabilisatoren kann dabei je nach bestimmter Gewebekonsistenz und Größe des Gliedma-ßenstumpfes angepasst werden. Im Mittel kann die Dicke der Stabilisatoren ≥ 3 mm und/oder ≤ 5 mm, vorzugsweise 4 mm und die Breite oder Länge ≥ 3 cm und/oder ≤ 5 cm, vorzugsweise 4 cm betragen.

Die Erfindung umfasst weiterhin ein Verfahren zur Herstellung eines Prothesenschafts, wobei zunächst mittels des vorbeschriebenen Verfahrens ein Modell für einen Gliedmaßenstumpf hergestellt wird und der Prothesenschaft auf Grundlage des hergestellten Modells hergestellt wird. Insbesondere kann zunächst in einem Prothesenschaftmodellerstellungsschritt auf Grundlage des mittels des vorbeschriebenen Verfahrens erstellten Modells für den Gliedma-ßenstumpf ein Prothesenschaftmodell erstellt werden und der Prothesenschaft auf Grundlage des Prothesenschaftmodells, vorzugsweise mittels eines additiven Fertigungsverfahrens, hergestellt werden.

Für den Prothesenschaft kann eine Schafteintrittsebene unter Berücksichtigung der Lage der Knochen konstruiert werden, insbesondere unter Verwendung des Knochenmodells, und gegebenenfalls in das Prothesenschaftmodell eingefügt werden.

Im Folgenden wird ein erfindungsgemäßes Verfahren zur Erstellung eines Modells für einen Gliedmaßenstumpf zur Herstellung eines Prothesenschafts anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

### Es zeigen

- Figur 1: ein 3d-Modell eines Oberschenkelstumpfes in einer Seitenansicht nach einem Modellerstellungsschritt gemäß einem Ausführungsbeispiel der Erfindung,
- Figur 2: das Oberschenkelstumpfmodell aus Figur 1 nach einem Einteilungsschritt gemäß dem Ausführungsbeispiel,
- Figur 3: das vorgenannte Oberschenkelstumpfmodell nach einem Modifikationsschritt gemäß dem Ausführungsbeispiel,
- Figur 4: das Oberschenkelstumpfmodell aus Figur 3 in einer Vorderansicht,
- Figur 5: das Oberschenkelstumpfmodell mit Knochenmodell in einer Vorderansicht gemäß dem Ausführungsbeispiel,
- Figur 6: das Oberschenkelstumpfmodell in einer Vorderansicht nach einem weiteren Modifikationsschritt gemäß dem Ausführungsbeispiel.

Im Folgenden wird ein Ausführungsbeispiel eines erfindungsgemäßen Verfahrens zur Erstellung eines Modells für einen Gliedmaßenstumpf zur Herstellung eines Prothesenschaftes für eine Beinprothese beschrieben. Dazu wird im Wesentlichen die Erstellung eines Modells für einen Oberschenkelstumpf beschrieben. Gemäß dem Ausführungsbeispiel kann das erfindungsgemäße Verfahren einen Scanschritt zum Scannen und Erfassen der Oberfläche des Oberschenkelstumpfes, einen Bestimmungsschritt zum Bestimmen einer Gewebekonsistenz des Oberschenkelstumpfes, einen Modellerstellungsschritt zum Erstellen eines Modells des Oberschenkelstumpfes auf Grundlage von in dem Scanschritt erfassten Daten, einen Einteilungsschritt zum Festlegen einer Bezugsebene in dem Modell und Einteilen des Modells in mehrere parallel zur Bezugsebene verlaufende Volumenscheiben, einen Reduktionsschritt zum Ermitteln basierend auf der bestimmten Gewebekonsistenz eines reduzierten Volumenwertes von mindestens einer Volumenscheibe und einen Modifikationsschritt zum Reduzieren eines Volumens der mindestens einen Volumenscheibe gemäß dem reduzierten Volumenwert unter Beibehaltung einer Dicke der Volumenscheibe und einer Form einer parallel zur Bezugsebene verlaufenden Querschnittskontur der Volumenscheibe und zum Anpassen des Modells entsprechend der volumenreduzierten Volumenscheibe.

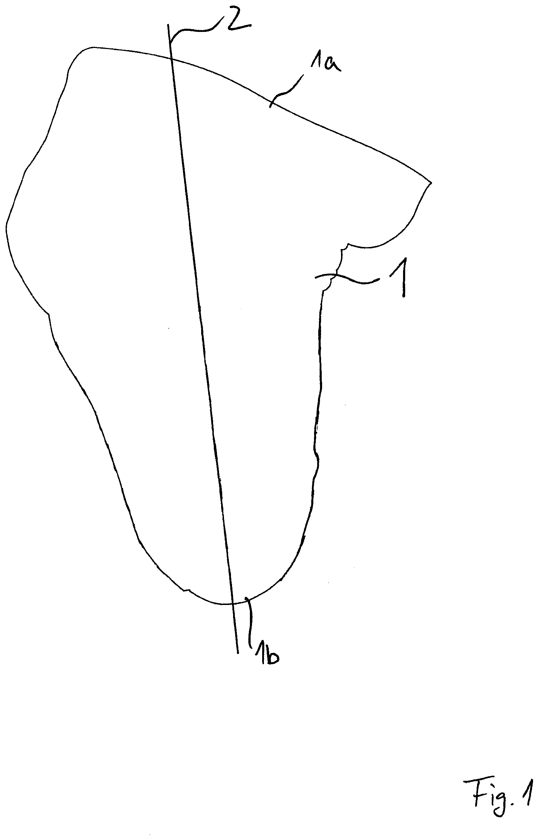

Figur 1 zeigt ein 3d-Modell eines Oberschenkstumpfes 1 in einer lateralen Seitenansicht. Das Modell ist aufgrund von Scandaten erstellt worden. Zum Scannen des Oberschenkelstumpfes wird dieser in eine spezielle Haltevorrichtung eingespannt, um eine reale Form des Oberschenkelstumpfes zu simulieren, wenn sich dieser in einer Prothese und/oder unter Last befindet. Der Oberschenkelstumpf wird weiterhin in der Seitenansicht und der Vorderansicht gescannt. Das gezeigte Oberschenkelstumpfmodell reicht in etwa von einem oberen, einem Hüftgelenk benachbarten Bereich 1a bis zu einem distalen, dem oberen Bereich 1a gegenüberliegenden Bereich 1b. Weiterhin ist eine Längsmittelachse 2 durch den Oberschenkelstumpf 1 in das Modell eingezeichnet. Zur Vereinfachung der nachträglichen Positionierung der Längsmittelachse 2 kann der Oberschenkelstumpf vor dem Scannen mit Markierungen, beispielsweise mittels Landmarks oder Hautmarkern, für die Längsmittelachse 2 versehen werden. Anhand der Längsmittelachse 2 wird im Einteilungsschritt die Bezugsebene zur Einteilung in Volumenscheiben festgelegt.

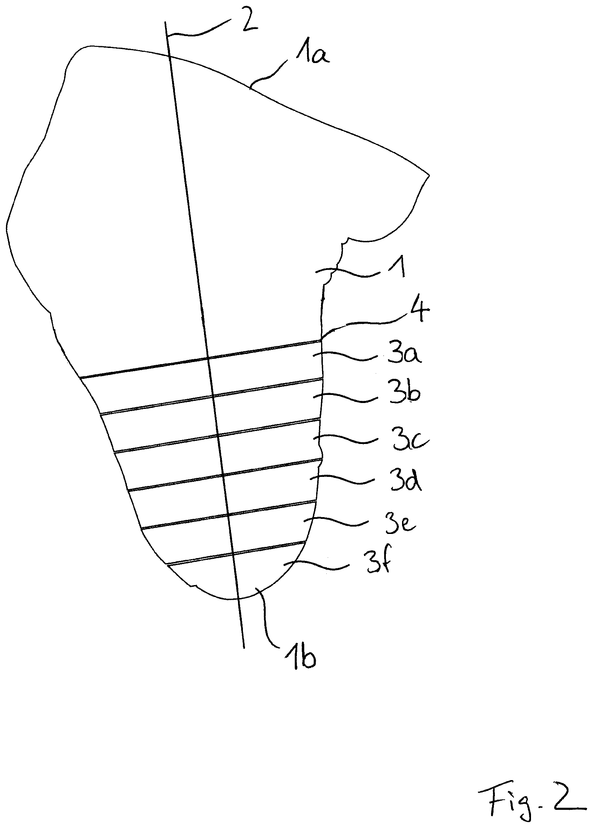

Figur 2 zeigt das Oberschenkelstumpfmodell nach dem Einteilungsschritt. Als Bezugsebene wird hier eine Ebene senkrecht zur Längsmittelachse 2 festgelegt. Parallel zu dieser Bezugsebene wird der Oberschenkelstumpf in mehrere Volumenscheiben 3a-3f gleicher Dicke von 3 cm eingeteilt. Dabei entspricht die Längsmittelachse 2 des Oberschenkelstumpfes auch etwa den Längsmittelachsen der einzelnen Volumenscheiben 3a-3f. Die oberste, also dem oberen Bereich 1a nächstgelegene Volumenscheibe 3a befindet sich direkt unterhalb einer Nullebene 4. Die Nullebene 4 wird dabei so gewählt, dass diese bei dem auf Grundlage des Modells hergestellten Prothesenschaft auf einer Höhe mit dem Sitzbeinhöcker (Tuber ischiadicum) und/oder dem Schambein (Ramus ischiadicum) liegt. Von jeder Volumenscheibe 3a-3f wird das Volumen bestimmt.

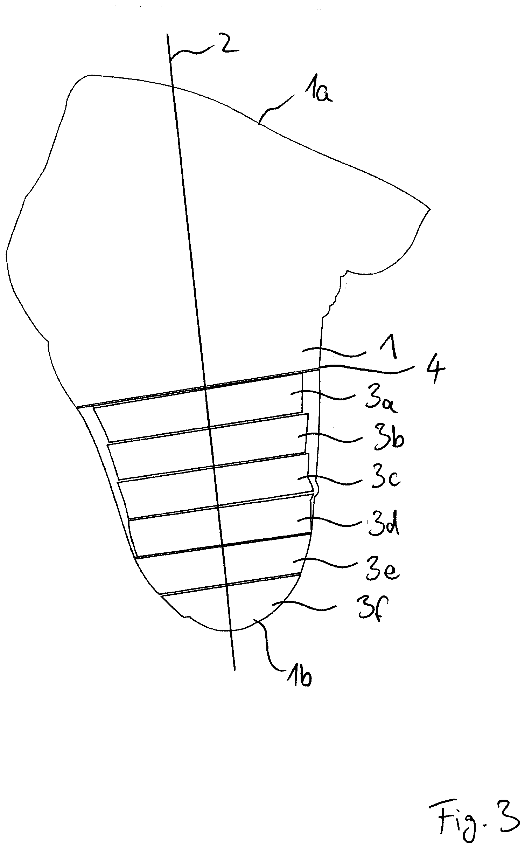

Figur 3 zeigt das Oberschenkelstumpfmodell 1 nach dem Reduktionsschritt. Anhand der bestimmten Gewebekonsistenz wird ein Faktor für die Volumenreduzierung jeder Volumenscheibe 3a-3f bestimmt. Mittels des Faktors wird dann aus dem ursprünglichen Volumen der Volumenscheibe der reduzierte Volumenwert der Volumenscheibe bestimmt. Im vorliegenden Ausführungsbeispiel können drei verschiedene Gewebekonsistenzen (fest, mittel, weich) bestimmt werden.

Alternativ oder ergänzend zur Bestimmung der Gewebekonsistenz als fest, mittel oder weich kann eine Kompressibilität des Gewebes mittels eines Zugbandmaßes gemessen werden. Die Zugbandmessung wird vorzugsweise entlang der obersten Volumenscheibe 3a sowohl im entspannten Zustand als auch unter Muskelanspannung durchgeführt und ein Mittelwert beider Messungen bestimmt, welcher in die Bestimmung der reduzierten Volumenwerte eingeht. Zur weiteren Optimierung der Passform können jedoch auch mehrere Zugbandmessungen entlang mehrerer Volumenscheiben durchgeführt werden.

Alternativ oder ergänzend zur Bestimmung der Gewebekonsistenz und zur Kompressibilitätsmessung können in die Ermittlung der reduzierten Volumenwerte auch Daten aus einer Sonografiemessung eingehen. Mittels der Sonografiemessung kann eine Verteilung verschiedener Gewebearten, wie beispielsweise Fettgewebe, Muskelgewebe und Knochengewebe, bestimmt werden. Beispielsweise kann ein Verlauf der Muskeln und von Muskellücken innerhalb des Stumpfes sowie eine Lage der Knochen erfasst werden.

Anhand der Daten aus der Zugbandmessung und/oder der Sonografiemessung kann der Faktor der Volumenreduktion der verschiedenen Volumenscheiben bestimmt werden. Die Volumenreduktion wird dabei so gewählt, dass die Reduktion der Volumenwerte desto stärker ist, je weicher bzw. kompressibler ein Gewebe ist. Die Volumenscheiben werden unter Beibehaltung der Dicken der Volumenscheiben und der Form der Querschnittskontur um die Längsmittelachse reduziert. In Figur 3 ist zu erkennen, dass in einem oberen Bereich des Oberschenkelstumpfes, welcher ein Bereich mit hohem Fettanteil ist, eine stärkere Volumenreduktion erforderlich ist als in einem distalen Bereich mit geringem Fettanteil. So erfolgt unterhalb der Nullebene 4 die stärkste Reduktion bei der Volumenscheibe 3a und fällt in Richtung distal immer geringer aus, wobei am Stumpfende 1b bei den Volumenscheiben 3e und 3f gar keine Volumenreduktion erfolgt.

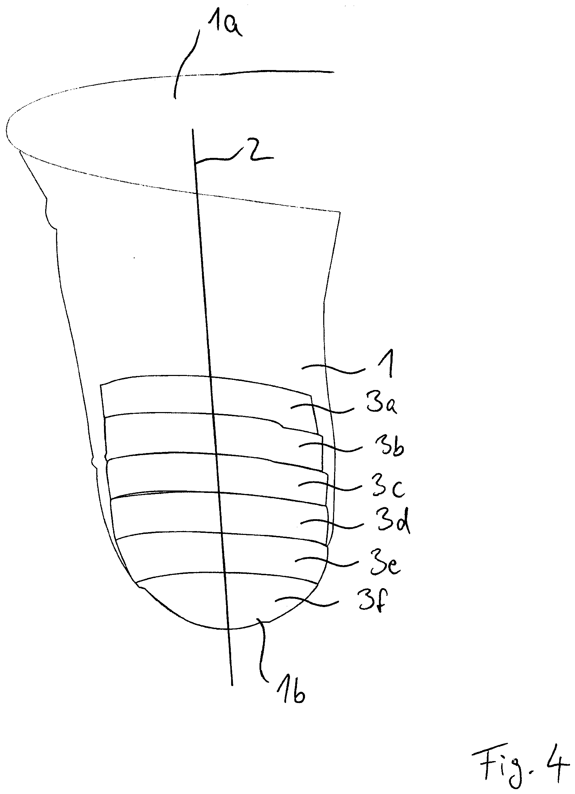

Figur 4 zeigt das Oberschenkelstumpfmodell in einer Vorderansicht nach der in Figur 3 bereits dargestellten Reduktion der Volumenwerte. Auch in der Vorderansicht ist erkennbar, dass eine Reduktion der Volumina der Volumenscheiben 3a-3d erfolgt, wobei die Stärke der Reduktion in Richtung distal abnimmt. Die Volumenscheiben 3e und 3f werden nicht reduziert.

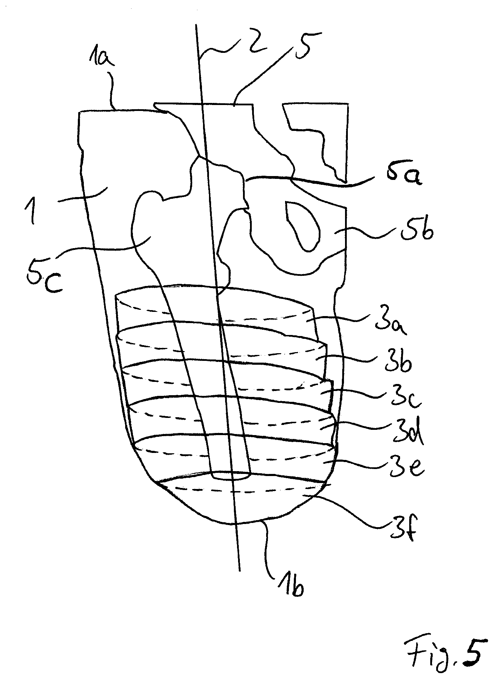

Figur 5 zeigt das Oberschenkelstumpfmodell 1 nach dem Reduktionsschritt in einer Vorderansicht. Weiterhin ist in das Oberschenkelstumpfmodell 1 ein Knochenmodell 5 eingepasst. Bei dem Knochenmodell handelt es sich um ein standardisiertes Modell, welches in seiner Größe und seinen Proportionen an den speziellen Stumpf angepasst werden kann. Im vorliegenden Fall umfasst das Knochenmodell 5 im Wesentlichen Knochen des Hüftgelenks 5a, also einen Teil des Beckengürtels 5b und einen oberen Teil des Oberschenkelknochens 5c. Das Knochenmodell 5 wird derart in das Oberschenkelstumpfmodell 1 eingepasst, dass der Oberschenkelknochen 5c im Wesentlichen entlang der Längsmittelachse 2 verläuft.

Im vorliegenden Fall wird die Einpassung des Knochenmodells 5 in das Oberschenkelstumpfmodell 1 nach dem Reduktionsschritt beschrieben. Das Knochenmodell 5 kann jedoch auch zu jedem anderen Zeitpunkt zwischen dem Scanschritt und der Fertigstellung des Oberschenkelstumpfmodells 1 oder der Weiterverarbeitung des Oberschenkelstumpfmodells 1 zur Herstellung des Prothesenschafts eingefügt werden.

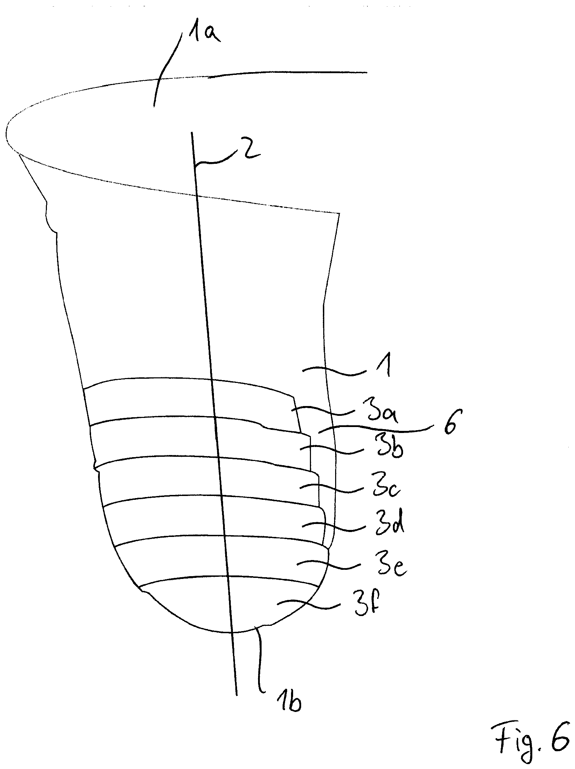

Figur 6 zeigt das Oberschenkelstumpfmodell 1 in einer Vorderansicht nach einem weiteren Teilschritt des Modifikationsschritts, in welchem die Volumenscheiben 3a-3d innerhalb ihrer Scheibenebene, also senkrecht zur Längsmittelachse 2, verschoben werden. Zur Vereinfachung der Abbildung wurde das Knochenmodell aus der Figur 6 herausgelassen. Im vorliegenden Fall erfolgt die Verschiebung der Volumenscheiben 3a-3d nach lateral, also an einen Außenrand des Oberschenkelstumpfmodells 1, sodass die Volumenscheiben 3a-3d bündig mit dem Außenrand des Oberschenkelstumpfmodells 1 sind und sich medial, also an einem Innenrand des Oberschenkelstumpfmodells 1, ein Raum 6 ergibt. Die Konturfläche des Oberschenkelstumpfmodells 1 wird dann am Innenrand an die Volumenscheiben 3a-3d angeglichen, sodass diese auch am Innenrand bündig mit der Konturfläche des Oberschenkelstumpfmodells 1 sind und der Raum 6 verschwindet. Dies sorgt dafür, dass der dem Oberschenkelstumpfmodell 1 entsprechende Prothesenschaft so geformt wird, dass dieser im Bereich seines Innenrandes näher an die Längsmittelachse 2 des Oberschenkelstumpfmodells 1 heranreicht, um eine mediale Abstützung des Oberschenkelstumpfes zu verbessern.

Nach der Volumenreduktion und der Verschiebung der Volumenscheiben 3a-3d werden die Volumenscheiben 3a-3d in dem Oberschenkelstumpfmodell 1 miteinander verbunden und die Konturfläche des Oberschenkelstumpfmodells 1 geglättet. Nach Fertigstellung des Oberschenkelstumpfmodells 1 kann das Oberschenkelstumpfmodell 1 zur Herstellung des Prothesenschaftes weiterverarbeitet werden.

## Patentansprüche

1. Verfahren zur Erstellung eines Modells für einen Gliedmaßenstumpf zur Herstellung eines Prothesenschafts, wobei
in einem Erfassungsschritt die Oberfläche des Gliedmaßenstumpfes erfasst wird,
in einem Bestimmungsschritt eine Gewebekonsistenz des Gliedmaßenstumpfs bestimmt wird und
in einem Modellerstellungsschritt ein Modell (1) des Gliedmaßenstumpfes
auf Grundlage von in dem Erfassungsschritt erfassten Daten erstellt wird,
in einem Einteilungsschritt in dem Modell eine Bezugsebene festgelegt wird und das Modell in mehrere parallel zur Bezugsebene verlaufende Volumenscheiben (3a-3f) eingeteilt wird,
in einem Reduktionsschritt basierend auf der bestimmten Gewebekonsistenz ein reduzierter Volumenwert von mindestens einer Volumenscheibe ermittelt wird, **dadurch gekennzeichnet, dass**
in einem Modifikationsschritt ein Volumen der mindestens einen Volumenscheibe gemäß dem reduzierten Volumenwert unter Beibehaltung einer Dicke der Volumenscheibe und einer Form einer parallel zur Bezugsebene verlaufenden Querschnittskontur der Volumenscheibe reduziert wird und das Modell entsprechend der volumenreduzierten Volumenscheibe angepasst wird.

2. Verfahren nach dem vorhergehenden Anspruch, wobei sich die Bezugsebene im Wesentlichen senkrecht zu einer Längsachse oder einer Längsmittelachse (2) des Gliedmaßenstumpfes erstreckt.

3. Verfahren nach einem der vorhergehenden Ansprüche, wobei in einem Sonografieschritt eine Verteilung verschiedener Gewebearten innerhalb des Gliedmaßenstumpfes bestimmt wird, und in dem Reduktionsschritt die reduzierten Volumenwerte basierend auf den im Sonografieschritt erfassten Daten ermittelt werden.

4. Verfahren nach einem der vorhergehenden Ansprüche, wobei die Volumenscheiben eine einheitliche oder unterschiedliche Dicke, bevorzugt eine Dicke von 3 cm aufweisen.

5. Verfahren nach einem der vorhergehenden Ansprüche, wobei das Modell weiterhin auf Grundlage eines Knochenmodells (5) für den Gliedmaßenstumpf erstellt wird oder ein Knochenmodell für den Gliedmaßenstumpf in das Modell eingepasst wird.

6. Verfahren nach dem vorhergehenden Anspruch, wobei das Knochenmodell ein in seiner Größe, Position und/oder seinen Proportionen veränderbares, standardisiertes oder individuelles Knochenmodell ist.

7. Verfahren nach einem der vorhergehenden Ansprüche, wobei eine Kompressibilität eines Stumpfgewebes gemessen wird, und die Kompressibilität bei der Ermittlung des reduzierten Volumenwertes berücksichtigt wird.

8. Verfahren nach einem der vorhergehenden Ansprüche, wobei in dem Modifikationsschritt eine Konturfläche des Modells des Gliedmaßenstumpfs in Abhängigkeit von einer Stärke der Reduktion des Volumens der mindestens einen Volumenscheibe zu einem Stumpfende hin verlängert wird.

9. Verfahren nach einem der vorhergehenden Ansprüche, wobei in dem Modifikationsschritt die volumenreduzierte Volumenscheibe parallel zur Bezugsebene und vorzugsweise innerhalb einer Konturfläche des Modells des Gliedmaßenstumpfs nach biomechanischen Aspekten, vorzugsweise nach lateral, verschoben wird und das Modell an die verschobene Volumenscheibe angepasst wird.

10. Verfahren nach einem der vorhergehenden Ansprüche, wobei in dem Modifikationsschritt ein distaler Anteil des Modells des Gliedmaßenstumpfs zur Entlastung des distalen Femurendes, vorzugsweise nach lateral, verkippt wird.

11. Verfahren nach einem der vorhergehenden Ansprüche, wobei in dem Modifikationsschritt entlang einer Umfangsrichtung einer Konturfläche des Modells Stabilisatoren zur Fixierung des Prothesenschafts in einer Umfangsrichtung eingefügt werden.

12. Verfahren zur Herstellung eines Prothesenschafts, wobei zunächst ein Modell für einen Gliedmaßenstumpf mittels eines Verfahrens nach einem der Ansprüche 1 bis 11 erstellt wird und anschließend der Prothesenschaft auf Grundlage des hergestellten Modells für einen Gliedmaßenstumpf, vorzugsweise mittels eines additiven Fertigungsverfahrens, hergestellt wird.

13. Verfahren nach einem dem vorhergehenden Anspruch, wobei für den Prothesenschaft eine Schafteintrittsebene unter Berücksichtigung der Lage der Knochen konstruiert wird, insbesondere unter Verwendung des Knochenmodells.

## Claims

1. A method of creating a model for a limb stump for the manufacture of a prosthesis stem, wherein
in a detection step, the surface of the limb stump is detected,
in a determination step, a tissue consistency of the limb stump is determined, and
in a model creation step, a model (1) of the limb stump is created based on data acquired in the detection step,
in a subdivision step, a reference plane is defined in the model and the model is subdivided into several volume slices (3a-3f) running parallel to the reference plane,
in a reduction step, a reduced volume value of at least one volume slice is determined based on the determined tissue consistency, **characterized in that**
in a modification step, a volume of the at least one volume slice is reduced according to the reduced volume value while maintaining a thickness of the volume slice and a shape of a cross-sectional contour of the volume slice extending parallel to the reference plane, and the model is adapted according to the volume-reduced volume slice.

2. The method according to the preceding claim, wherein the reference plane extends substantially perpendicular to a longitudinal axis or a longitudinal central axis (2) of the limb stump.

3. The method according to any one of the preceding claims, wherein in a sonography step, a distribution of different tissue types within the limb stump is determined, and in the reduction step, the reduced volume values are determined based on the data acquired in the sonography step.

4. The method according to any one of the preceding claims, wherein the volume slices have a uniform or different thickness, preferably a thickness of 3 cm.

5. The method according to any one of the preceding claims, wherein the model is further created based on a bone model (5) for the limb stump or a bone model for the limb stump is fitted into the model.

6. The method according to the preceding claim, wherein the bone model is a standardized or customized bone model that can be changed in size, position and/or proportions.

7. The method of any one of the preceding claims, wherein a compressibility of a limb tissue is measured, and the compressibility is taken into account in determining the reduced volume value.

8. The method of any one of the preceding claims, wherein in the modification step, a contour surface of the model of the limb stump is extended toward a stump end in response to an amount of reduction in volume of the at least one volume slice.

9. The method according to any of the preceding claims, wherein in the modification step, the volume-reduced volume slice is displaced parallel to the reference plane and preferably within a contour surface of the model of the limb stump according to biomechanical aspects, preferably laterally, and the model is adapted to the displaced volume slice.

10. The method according to any one of the preceding claims, wherein in the modification step, a distal portion of the model of the limb stump is tilted, preferably laterally, to relieve the distal end of the femur.

11. The method according to any one of the preceding claims, wherein in the modification step, stabilizers are inserted along a circumferential direction of a contour surface of the model to fix the prosthesis stem in a circumferential direction.

12. A method of manufacturing a prosthesis stem, wherein a model for a limb stump is first created by means of a method according to any one of claims 1 to 11, and subsequently the prosthesis stem is manufactured on the basis of the created model for a limb stump, preferably by means of an additive manufacturing process.

13. The method according to any one of the preceding claims, wherein a stem entry plane is constructed for the prosthesis stem taking into account the position of the bones, in particular using the bone model.

## Revendications

1. Procédé de création pour un modèle de moignon de membre pour la production d'une tige de prothèse, dans lequel
dans une étape d'acquisition, la surface du moignon de membre est acquise,
dans une étape de détermination, une consistance de tissu du moignon de membre est déterminée et
dans une étape de création de modèle, un modèle (1) du moignon de membre est créé sur la base des données acquises dans l'étape d'acquisition,
dans une étape de division, un plan de référence est défini dans le modèle et le modèle est divisé en plusieurs tranches de volume (3a-3f) s'étendant parallèlement au plan de référence,
dans une étape de réduction, une valeur de volume réduite d'au moins une tranche de volume est établie sur la base de la consistance de tissu déterminée, **caractérisé en ce que**,
dans une étape de modification, un volume de la au moins une tranche de volume est réduit conformément à la valeur de volume réduite en conservant une épaisseur de la tranche de volume et une forme d'un contour de section transversale de la tranche de volume s'étendant parallèlement au plan de référence et le modèle est adapté en fonction de la tranche de volume réduite en volume.

2. Procédé selon la revendication précédente, dans lequel le plan de référence s'étend sensiblement perpendiculairement à un axe longitudinal ou un axe médian longitudinal (2) du moignon de membre.

3. Procédé selon l'une quelconque des revendications précédentes, dans lequel, dans une étape d'échographie, une répartition de différents types de tissus à l'intérieur du moignon de membre est déterminée, et dans l'étape de réduction, les valeurs de volume réduites sont établies sur la base des données acquises dans l'étape d'échographie.

4. Procédé selon l'une quelconque des revendications précédentes, dans lequel les tranches de volume ont une épaisseur uniforme ou différente, de préférence une épaisseur de 3 cm.

5. Procédé selon l'une quelconque des revendications précédentes, dans lequel le modèle est en outre créé sur la base d'un modèle d'os (5) pour le moignon de membre ou un modèle d'os pour le moignon de membre est intégré dans le modèle.

6. Procédé selon la revendication précédente, dans lequel le modèle d'os est un modèle d'os standardisé ou individuel dont la taille, la position et/ou les proportions peuvent être modifiées.

7. Procédé selon l'une quelconque des revendications précédentes, dans lequel une compressibilité d'un tissu de moignon est mesurée, et la compressibilité est prise en compte lors de l'établissement de la valeur de volume réduit.

8. Procédé selon l'une quelconque des revendications précédentes, dans lequel, dans l'étape de modification, une surface de contour du modèle du moignon de membre est allongée vers une extrémité du moignon en fonction d'une intensité de la réduction du volume de la au moins une tranche de volume.

9. Procédé selon l'une quelconque des revendications précédentes, dans lequel, dans l'étape de modification, la tranche de volume réduite en volume est déplacée parallèlement au plan de référence et de préférence à l'intérieur d'une surface de contour du modèle du moignon de membre selon des aspects biomécaniques, de préférence latéralement, et le modèle est adapté à la tranche de volume déplacée.

10. Procédé selon l'une quelconque des revendications précédentes, dans lequel, dans l'étape de modification, une partie distale du modèle du moignon de membre est basculée, de préférence latéralement, pour soulager l'extrémité distale du fémur.

11. Procédé selon l'une quelconque des revendications précédentes, dans lequel, dans l'étape de modification, des stabilisateurs sont insérés le long d'une direction circonférentielle d'une surface de contour du modèle pour fixer la tige de prothèse dans une direction circonférentielle.

12. Procédé de production d'une tige de prothèse, dans lequel un modèle pour un moignon de membre est d'abord créé au moyen d'un procédé selon l'une quelconque des revendications 1 à 11 puis la tige de prothèse est produite sur la base du modèle produit pour un moignon de membre, de préférence au moyen d'un procédé de fabrication additive.

13. Procédé selon l'une quelconque des revendications précédentes, dans lequel un plan d'entrée de tige est construit pour la tige de prothèse en tenant compte de la position des os, en particulier en utilisant le modèle d'os.
